(19) Europäisches Patentamt
European Patent Office
Office européen des brevets

(11) Veröffentlichungsnummer: **0 074 124**
**A1**

(12) # EUROPÄISCHE PATENTANMELDUNG

(21) Anmeldenummer: 82108529.7

(22) Anmeldetag: 08.10.79

(51) Int. Cl.³: **A 01 N 41/06**

(30) Priorität: 23.10.78 DE 2845997

(43) Veröffentlichungstag der Anmeldung: 16.03.83 Patentblatt 83/11

(84) Benannte Vertragsstaaten: BE CH DE FR GB IT NL

(60) Veröffentlichungsnummer der früheren Anmeldung nach Art. 76 EPÜ: 0 010 250

(71) Anmelder: BAYER AG
Zentralbereich Patente, Marken und Lizenzen
D-5090 Leverkusen 1, Bayerwerk(DE)

(72) Erfinder: Kühle, Engelbert, Dr.
von-Bodelschwingh-Strasse 42
D-5060 Bergisch-Gladbach 2(DE)

(72) Erfinder: Lürssen, Klaus, Dr.
August-Kierspel-Strasse 89
D-5060 Bergisch-Gladbach(DE)

(54) **Verwendung von Chlormethansulfonsäure-2-trifluormethyl-4-methylthio-anilid zur Regulierung des Pflanzenwachstums.**

(57) Das bekannte Chlormethansulfonsäure-2-trifluormethyl-4-methylthio-anilid der Formel

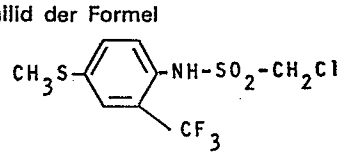 (1)

ist sehr gut zur Regulierung des Pflanzenwachstums geeignet.

EP 0 074 124 A1

0074124

BAYER AKTIENGESELLSCHAFT 5090 Leverkusen, Bayerwerk

Zentralbereich
Patente, Marken und Lizenzen Dü-klu.
IIb

# Verwendung von Chlormethansulfonsäure-2-trifluormethyl-4-methylthio-anilid zur Regulierung des Pflanzenwachstums

Die vorliegende Anmeldung betrifft die Verwendung des bekannten Chlormethansulfonsäure-2-trifluormethyl-4-methylthioanilids als Wirkstoff zur Regulierung des Pflanzenwachstums.

Es ist bekannt geworden, daß 2-Chlorethylphosphonsäure pflanzenwuchsregulierende Eigenschaften aufweist (vgl. Deutsche Offenlegungsschrift 2 050 245). Die Wirksamkeit dieses Stoffes ist jedoch, - vor allem bei niedrigen Aufwandmengen -, nicht immer ganz befriedigend.

Es wurde nun gefunden, daß das bekannte Chlormethansulfonsäure-2-trifluormethyl-4-methylthio-anilid der Formel

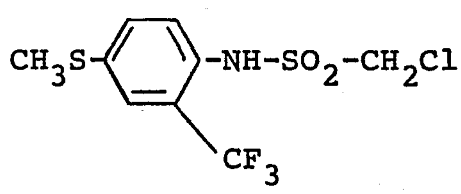

(I)

sehr gut zur Regulierung des Pflanzenwachstums geeignet ist.

Überraschenderweise zeigt das erfindungsgemäß verwenbare Sulfonanilid der Formel (I) eine wesentlich bessere pflanzenwuchsregulierende Wirksamkeit als die aus dem Stand der Technik bekannte 2-Chlorethylphosphonsäure, welches ein hochaktiver Wirkstoff gleicher Wirkungsart ist. Der erfindungsgemäß verwendbare Stoff stellt somit eine wertvolle Bereicherung der Technik dar.

Das erfindungsgemäß verwendbare Chlormethansulfonsäure-2-trifluormethyl-4-methylthio-anilid der Formel (I) ist bereits bekannt (vgl. DE-A 27 03 477).

Der erfindungsgemäß verwendbare Wirkstoff greift in den Metabolismus der Pflanzen ein und kann deshalb als Wachstumsregulator eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch

vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Sulfonanilide zeichnen sich nicht nur durch eine gute pflanzenwachstumsregulierende Wirksamkeit aus, sondern eignen sich auch als Entwicklungshemmer bei Insekten.

Der Wirkstoff kann in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton,

Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Äther, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und

Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäß verwendbare Wirkstoff kann in den Formulierungen in Mischung mit anderen bekannten Wirkstoffen vorliegen, wie Fungizide, Insektizide; Akarizide und Herbizide, sowie in Mischungen mit Düngemitteln und anderen Wachstumsregulatoren.

Der Wirkstoff kann als solcher, in Form seiner Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Begasen usw. Es ist ferner möglich, den Wirkstoff nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung des Wachstumsregulators in einem bevorzugten Zeitraum vorgenommen wird, dessen genaue Abgrenzung sich nach dem klimatischen und vegetativen Gegebenheiten richtet.

Im folgenden wird die Aktivität des erfindungsgemäß verwendbaren Sulfonanilides der Formel (I) als Pflanzenwachstumsregulator durch einige Beispiele illustriert.

Beispiel A

Wuchshemmung bei Weizen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum 2-Blatt-Stadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in % des Zuwachses der Kontrollpflanzen berechnet. Es bedeuten 100 % den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

<u>Tabelle A</u>

Wuchshemmung bei Weizen

| Wirkstoff | Wirkstoffkonzentration in % | Wuchshemmung in % |
|---|---|---|
| - (Kontrolle) | - | 0 |
| 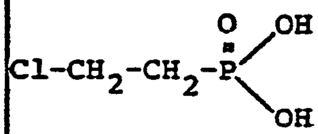 (bekannt) | 0,05 | 0 |
| 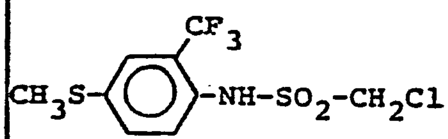 | 0,05 | 85 *) **) |

*) dunkelgrüne Blattfarbe

**) Bestockung

## Beispiel B

### Wuchshemmung bei Sojabohnen

Lösungsmittel: 3o Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 1oo % den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

## Tabelle B

Wuchshemmung bei Sojabohnen

| Wirkstoff | Wirkstoffkonzentration in % | Wuchshemmung in % |
|---|---|---|
| - (Kontrolle) | - | 0 |
| 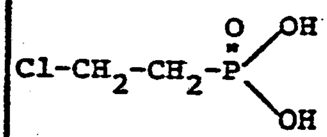 (bekannt) | 0,05 | 50 |
| 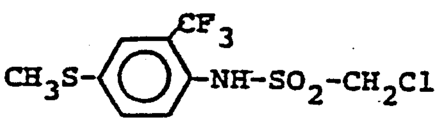 | 0,05 | 85 |

0074124

Beispiel C

Wuchshemmung bei Gerste

Lösungsmittel: 3o Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% den Stillstand des Wachstums und 0% ein Wachstum entsprechend dem der Kontrollpflanzen.

Wirkstoffe, Wirkstoffkonzentrationen und Resultate gehen aus der nachfolgenden Tabelle hervor.

Tabelle C

Wuchshemmung bei Gerste

| Wirkstoff | Wirkstoffkonzentration in % | Wuchshemmung in % |
|---|---|---|
| - (Kontrolle) | - | 0 |
| 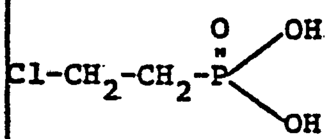 (bekannt) | 0,05 | 0 |
| 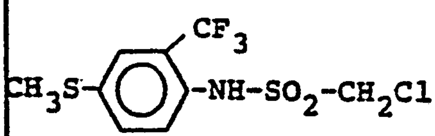 | 0,05 | 45 *) **) |

*) dunkelgrüne Blattfarbe

**) Bestockung

Herstellungsbeispiel

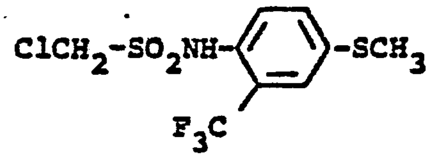

31,5 g (0.153 Mol) 2-Trifluormethyl-4-methylthioanilin werden in 100 ml Pyridin gelöst und bei 0°C bis 10°C tropfenweise mit 56,6 g (0.38 Mol) Chlormethansulfochlorid versetzt. Man rührt eine Stunde bei Raumtemperatur nach, trägt dann die Reaktionslösung in verdünnte Salzsäure ein und extrahiert mit Methylenchlorid. Nach dem Trocknen der organischen Phase wird das Lösungsmittel abgezogen. Es verbleiben 50 g eines dunklen Öles, das im wesentlichen aus N,N-(Bischlormethylsulfonyl)-2-trifluormethyl-4-methylthioanilin besteht.

42 g des Öles werden in 200 ml Methanol gelöst. Diese Lösung wird mit 17 g (0.3 Mol) Kaliumhydroxid versetzt und anschließend einige Stunden bei Raumtemperatur gerührt. Nach einiger Zeit beginnt sich ein kristallines Produkt abzuscheiden. Man löst dieses in Wasser und säuert mit Salzsäure an. Hierbei erhält man 28 g ( 90 % der Theorie) an Chlormethansulfonsäure-2-trifluromethyl-4-methylthioanilid in Form einer Festsubstanz vom Schmelzpunkt 98-99°C.

Patentanspruch

Verwendung von Chlormethansulfonsäure-2-trifluormethyl-4-methylthioanilid der Formel

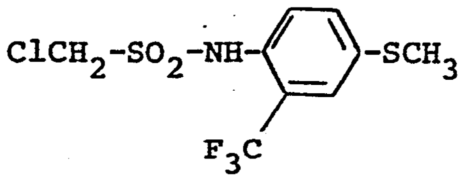

(I)

zur Regulierung des Pflanzenwachstums.

Europäisches Patentamt

# EUROPÄISCHER RECHERCHENBERICHT

0074124
Nummer der Anmeldung

EP 82 10 8529

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A-2 378 754 (BAYER)<br>* Seite 7, Zeilen 36-39 * | 1 | A 01 N 41/06 |
| | | | **RECHERCHIERTE SACHGEBIETE (Int. Cl. 3)** |
| | | | A 01 N |

Der vorliegende Recherchenbericht wurde für alle Patentansprüche erstellt.

| Recherchenort | Abschlußdatum der Recherche | Prüfer |
|---|---|---|
| DEN HAAG | 09-12-1982 | DECORTE D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82